## Europäisches Patentamt

## European Patent Office

## Office européen des brevets

(19)

(11) Veröffentlichungsnummer: **0 150 782**
**B1**

(12) **EUROPÄISCHE PATENTSCHRIFT**

(45) Veröffentlichungstag der Patentschrift: **04.07.90**

(21) Anmeldenummer: **85100543.9**

(22) Anmeldetag: **19.01.85**

(51) Int. Cl.⁵: **C 12 P 33/12, C 07 J 1/00 //
C07J53/00**

(54) **Verfahren zur Herstellung von 15alpha-Hydroxy-4-androsten-3,17-dion.**

(30) Priorität: **02.02.84 DE 3403862**

(43) Veröffentlichungstag der Anmeldung:
**07.08.85 Patentblatt 85/32**

(45) Bekanntmachung des Hinweises auf die
Patenterteilung:
**04.07.90 Patentblatt 90/27**

(84) Benannte Vertragsstaaten:
**AT BE CH DE FR GB IT LI LU NL SE**

(56) Entgegenhaltungen:
**US-A-2 889 255**

**RECUEIL DES TRAVAUX CHEMIQUES DES
PAYS-BAS, Band 82, Nr. 2, Februar 1963, Seiten
143-148, 's-Gravenhage, NL; J. DE FLINES et al.:
"Microbiological conversion of 19-
nortestosterone (IV)**

**Die Akte enthält technische Angaben, die nach
dem Eingang der Anmeldung eingereicht
wurden und die nicht in dieser Patentschrift
enthalten sind.**

(73) Patentinhaber: **SCHERING
AKTIENGESELLSCHAFT Berlin und Bergkamen
Müllerstrasse 170/178 Postfach 65 03 11
D-1000 Berlin 65 (DE)**

(72) Erfinder: **Petzoidt, Karl, Dr.
Flachsweg 10
D-1000 Berlin 38 (DE)**

(56) Entgegenhaltungen:

**HELVETICA CHIMICA ACTA, Band XLI, Nr. 1, 1.
Februar 1958, Seiten 301-305, Schweizerische
Chemische Gesellschaft, Basel, CH; A. GUBLER
et al.: "36. Hydroxylierung von Androsten-(4)-
dion-(3,17), Testosteron, Progesteron und
Cortexon durch Fusarium lini (Bolley)"
ENDOCRINOLOGY 83, 1968, S. 348
HELVETICA CHIMICA ACTA, Band XLVI, Fasc.III,
1963, S. 889
BIOCHEMISTRY, Band 11, Nr. 5, 1972, S. 753**

EP 0 150 782 B1

## Beschreibung

Es ist seit langem bekannt, daß zahlreiche Mikroorganismen die Fähigkeit besitzen, Steroide in der 15α-Position zu hydroxylieren (US-Patentschrift 2,889,255). Auch das 4-Androsten-3,17-dion wurde bereits mehrfach in der 15α-Position hydroxyliert. (W. Charney a. H.L. Herzog: Microbial Transformations of Steroids, Academic Press, New York, 1967, Seite 107,108). Soweit man der Literatur entnehmen kann, wurden dabei die besten Ergebnisse erzielt, wenn man zur Fermentation Mikroorganismen der Species Fusarium lini (CBS) (Helvetica Chimica Acta 46, 1963, 889 f) oder Penicillium spec (ATCC 11598) (Endocrinology 83, 1968, 349) verwendet. Für eine technische Herstellung von 15α-Hydroxy-4-androsten-3,17-dion sind diese bekannten Verfahren zu aufwendig, da bei ihnen nicht einemal 100 mg 4-Androsten-3,17-dion pro Liter Fermentationskultur und pro Tag Fermentationsdauer umgewandelt werden.

Aus der US-Patentschrift 4,036,695 ist andererseits bekannt, daß man das zum 4-Androsten-3,17-dion isomere 18-Methyl-4-estren-3,17-dion in hohen Substratkonzentrationen in der 15α-Position hydroxylieren kann, wenn man zur Fermentation Mikroorganismen der Species Penicillium raistrickii (ATCC 10490) verwendet. Überraschenderweise verläuft die Fermentation von 4-Androsten-3,17-dion mit diesem Mikroorganismus äußerst unbefriedigend.

Es wurde gefunden, daß man überraschenderweise 4-Androsten-3,17-dion recht gut in der 15α-Position hydroxylieren kann, wenn man zur Fermentation die im Patentanspruch genannten Mikroorganismen verwendet.

Diese Fermentation wird unter den Bedingungen durchgeführt, die man üblicherweise bei der mikrobiologischen Hydroxylierung von Steroiden mit Pilzkulturen anwendet. So werden zunächst in allgemein üblichen Vorversuchen die günstigsten Fermentationsbedingungen, wie zum Beispiel Auswahl des günstigsten Nährmediums, des geeigneten Substratlösungsoder Suspensionsmittels, der Substratkonzentration, der technischen Bedingungen wie Temperatur, Belüftung, pH-Wert und der optimalen Zeiten für Germination, Substratzugabe und Substratkontakt am Enzym des Mikroorganismus analytisch, insbesondere dünnschichtchromatographisch, ermittelt.

Es ist zweckmäßig, das Substrat in einer Konzentration von etwa 1000 bis 5000 mg pro Liter Nährmedium einzusetzen. Der pH-Wert wird vorzugsweise auf einen Bereich von 5 bis 7 eingestellt. Die Züchtungstemperatur liegt im Bereich von 20 bis 40°C, vorzugsweise von 25 bis 35°C. Zur Belüftung werden vorzugsweise 0,5 bis 5 Liter Luft pro Minute pro Liter Kulturbrühe zugeführt. Die Umwandlung des Substrats wird zweckmäßigerweise durch dünnschichtchromatographische Analyse verfolgt. Die Fermentationszeit beträgt etwa 30 bis 90 Stunden.

Das erhaltene 15α-Hydroxy-4-androsten-3,17-dion ist ein wertvolles Zwischenprodukt, welches sich beispielsweise zur Herstellung von 15β,16β-Methylen-4-androsten-3,17-dion (US-Patentschrift 3,470,160) oder von 15β,16β-Methylen-3-oxo-17α-pregna-4,6-dien-21,17-carbolacton (US-Patentschrift 4,129,564) eignet.

Die nachfolgenden Beispiele dienen zur näheren Erläuterung des erfindungsgemäßen Verfahrens und zur Verwendung des Verfahrensprodukts.

A. Beispiele zur Erläuterung des erfindungsgemäßen Verfahrens:

### Beispiel 1

Ein 2 l-Erlenmeyerkolben, der 500 ml einer 30 Minuten bei 120°C im Autoklaven sterilisierten Nährlösung enthaltend 3,0% Glucose, 1,0% Corn steep liquo 0,2% Natriumnitrat, 0,1% Kaliumdihydrogenphosphat, 0,2% Dikaliumhydrogenphosphat, 0,05% Magnesiumsulfat-Heptahydrat, 0,002% Eisen(II)-sulfat-Heptahydrat und 0,05% Kaliumchlorid wird mit einer Schrägröhrchen-Kultur des Stammes Penicillium stoloniferum (CBS P102) beimpft und 2 1/2 Tage bei 28°C auf einem Rotationsschüttler geschüttelt.

Mit 250 ml dieser Anzuchtkultur wird ein 20 l-Vorfermenter beimpft, der mit 15 l eines 60 Min. bei 121°C und 1,1 bar Überdruck sterilisierten Mediums, enthaltend 1,0% Corn steep liqour, 1,0% Stärkezucker, 1,0% Sojapuder, 0,2% Natriumnitrat, 0,1% Kaliumdihydrogenphosphat, 0,2% Dikaliumhydrogenphosphat, 0,05% Magnesiumsulfat-Heptahydrat, 0,002% Eisen(II)-sulfat-Heptahydrat und 0,05% Kaliumchlorid gefüllt ist.

Unter Zugabe von Silicon SH als Antischaummittel wird bei 29°C und 0,7 bar Überdruck unter Belüftung (15 l/Min.) und Rühren (220 U/Min.) 24 Stunden germiniert.

Danach werden 0,9 l dieser Kultur unter sterilen Bedingungen entnommen und damit ein 20 l-Hauptfermenter beimpft, der mit 14 l eines wie oben sterilisierten Nährmediums der gleichen Zusammensetzung wie die Vorfermenterkultur beschickt ist. Nach einer Anwachsphase von 12 Stunden unter Vorfermenterbedingungen wird eine sterilfiltrierte Losung von 22,5 g 4-Androsten-3,17-dion in 200 ml Dimethylformamid hinzugegeben und weiter gerührt und belüftet. Nach 60 Stunden Kontaktzeit wird die Kulturbrühe dreimal mit je 10 l Methylisobutylketon extrahiert, die Extrakte werden vereinigt und zunächst im Umlaufverdampfer konzentriert, anschließend bei 50°C Badtemperatur im Vakuum am Rotationsverdampfer zur Trockne eingeengt. Den Rückstand wäscht man zur Entfernung des Antischaummittels mit Hexan kristallisiert schließlich aus Methylisobutylketon. Nach dem Absaugen und Trocknen erhält man 18,4 g 15α-Hydroxy-4-androsten-3,17-dion vom Schmelzpunkt 193-196°C.

### Beispiel 2

Unter den Bedingungen des Beispiels 1 werden unter Verwendung des Stammes Penicillium melinii (CBS P 30) 15 g 4-Androsten-3,17-dion in 75 Stunden Kontaktzeit zu 11,7 g 15α-Hydroxy-4-androsten-3,17-dion vom Schmelzpunkt 191-194°C umgewandelt.

### Beispiel 3

Unter den Bedingungen des Beispiels 1 werden unter Verwendung des Stammes Fusarium oxysporum f. vasinfectum ATCC 7808 22, 5 g 4-Androsten-3,17-dion in 24 Stunden Kontaktzeit zu 19,36 g 15α-Hydroxy-4-androsten-3,17-dion vom Schmelzpunkt 197°C umgewandelt.

B. Beispiel zur Verwendung des 15α-Hydroxy-4-androsten-3,17-dions

a) Eine Lösung von 40 g 15α-Hydroxy-4-androsten-3,17-dion in 200 ml Pyridin wird im Eisbad abgekühlt und dann tropfenweise mit 25,7 ml Benzoylchlorid versetzt. Nach 30 Minuten Rühren bei Eisbadkühlung wird mit 3,2 ml Wasser versetzt und weitere 30 Minuten gerührt. Die Reaktionslösung wird dann in Eiswasser eingerührt, der ausgefallene Niederschlag wird abgesaugt und mit Wasser gewaschen. Dabei beginnt das Fällungsprodukt ölig zu werden, es wird daher in Methylenchlorid aufgenommen. Die organische Phase wird mit Wasser gewaschen, über Natriumsulfat getrocknet und eingedampft. Es werden 53 g rohes 15α-Benzoyloxy-4-androsten-3,17-dion als Öl erhalten.

b) 101,5 g Trimethylsulfoxoniumiodid werden in 1 l Dimethylsulfoxyd mit 17,88 g NaOH 1,5 Stunden bei Raumtemperatur kräftig gerührt, das NaOH ist dann weitgehend in Lösung gegangen. Unter Argon und Wasserkühlung werden dann 53 g 15α-Benzoyloxy-4-androsten-3,17-dion in 200 ml Dimethylsulfoxyd gelöst, innerhalb 5 Minuten in das Reaktionsgemisch eingetragen, das anschließend 1,5 Stunden nachgerührt wird. Die Reaktionslösung wird dann, unter Zusatz von ca. 2 kg NaCl, in die 10-fache Menge Eiswasser eingerührt. Der ausgefällte Niederschlag wird mit Wasser gewaschen und im Vakuum bei 50°C getrocknet. Man erhält 31 g der rohen Methylen-Verbindung 15β,16β-Methylen-4-androsten-3,17-dion. Die gesamte wässrige Phase wird mit Diethylether extrahiert, die Etherphase wird mit gesättigter NaCl-Lösung gewaschen, über Na$_2$SO$_4$ getrocknet und eingedampft. Es werden nochmals 6 g rohe Methylen-Verbindung 15β,16β-Methylen-4-androsten-3,17-dion erhalten. Niederschlag und Etherextrakt werden zusammengefaßt und an Kieselgel mit einem Aceton-Hexan-Gradienten (20 l, 0—25% Aceton) chromatographiert. Man eluiert 22,7 g 15β,16β-Methylen-4-androsten-3,17-dion. Eine aus Diisopropylether umkristallisierte Probe schmilzt bei 185-186,5°C.

c) Eine Lösung von 22,7 g 15β,16β-Methylen-4-androsten-3,17-dion in 397 ml Methylpyrrolidon wird im Eisbad abgekühlt, unter Argon mit 90,8 g Kaliumethylat versetzt und 10 Minuten gerührt. Dann werden innerhalb 5 Minuten 34,1 ml Propargylalkohol zugetropft. Das Reaktionsgemisch wird weitere 15 Minuten gerührt und in die 10-fache Menge Eiswasser eingerührt. Man neutralisiert mit verdünnter Schwefelsäure, entfernt einen eventuellen Überschuß mit NaHCO$_3$ und sättigt mit Natriumchlorid. Der ausgefallene Niederschlag wird abgesaugt, mit Wasser gewaschen und im Vakuum bei 60°C getrocknet. Man erhält 30,2 g, die aus Essigester mit Aktivkohle umkristallisiert werden. Ausbeute 18,6 g eines Gemisches aus 17β-Hydroxy-17α-(3-hydroxy-1-propionyl)-15β,16β-methylen-4-androsten-3-on und 17β-Hydroxy-17α-(3-hydroxy-1-propinyl)-15β,16β-methylen-5-androsten-3-on vom Schmelzpunkt 224,6°C.

Eine Lösung von 18,6 g dieses Gemisches in 172 ml Tetrahydrofuran und 172 ml Methanol wird unter Argon mit 8,6 ml 2N Natronlauge versetzt und 15 Minuten bei Raumtemperatur gerührt. Die Reaktionsmischung wird dann in die 10-fache Menge Eiswasser, das 10 ml 2N Schwefelsäure enthält, eingerührt. Man sättigt mit Kochsalz, saugt den ausgefällten Niederschlag ab und wäscht mit Wasser. Nach dem Trocknen bei 60°C im Vakuum werden 18,2 g 17β-Hydroxy-17α-(3-hydroxyl-propinyl)-15β,16β-methylen-4-androsten-3-on erhalten.
Schmelzpunkt 222,5°C.

d) 18,2 g 17β-Hydroxy-17α-(3-hydroxy-1-propinyl)-15β,16βmethylen-4-androsten-3-on werden in einem Gemisch aus 84 ml Tetrahydrofuran und 84 ml Methanol nach Zusatz von 5,6 g Tris(triphenyl-phosphin-rhodium(l)-chlorid bei Raumtemperatur und Normaldruck bis zur Sättigung mit Wasserstoff hydriert.
Reaktionszeit: 5 Stunden.

Die Substanz geht bei der Hydrierung vollständig in Lösung.

Anschließend wird die Hydrierlösung im Vakuum zur Trockne eingedampft. Das erhaltene Rohprodukt von 18,0 g 17β-Hydroxy-17α-(3-hydroxypropyl)-15β,16β-methylen-4-androsten-3-on wird zur nächsten Umsetzung direkt verwendet.

e) Eine Lösung von 18 g des 17β-Hydroxy-17α-(3-hydroxypropyl)-15β,16β-methylen-4-androsten-3-on Rohprodukt in 238 ml Pyridin wird mit einer Lösung von 47,6 g CrO3 in 118 ml Wasser und 119 ml Pyridin versetzt und 6 Stunden bei 70°C gerührt. Die Reaktionslösung wird anschließend in 4 l Essigester eingerührt. Man dekantiert von den ausgeschiedenen Chromsalzen ab und wäscht die Essigesterphase mit Wasser. Nach dem Trocknen und Eindampfen wird das pyridinhaltige Rohprodukt mit einem Aceton-Hexan-Gradienten (20 l, 0-40% Aceton) an Kieselgel chromatographiert Man erhält 11,3 g, die nach dem Verreiben mit Diisopropylether 9,72 g 15β,16β-Methylen-3-oxo-17α-pregn-4-en-21,17-carbolacton vom Schmelzpunkt 179-180°C ergeben.

f) Eine Lösung von 150 g 15β,16β-Methylen-3-oxo-17α-pregn-4-en-21,17-carbolacton in 1,5 l Dioxan wird mit 150 ml o-Ameisensäuretriethylester und 150 ml Dioxan/conc. Schwefelsäure versetzt und 1 Stunde bei Raumtemperatur gerührt. Die Reaktionslösung wird mit 13,5 ml Pyridin versetzt, mit 8,1 l Dichlormethan verdünnt, mit Wasser gewaschen, über Magnesiumsulfat getrocknet und im Vakuum eingedampft. Es werden 160 g kristallines 3-Ethoxy-15β,16β-methylen-17α-pregna-3,5-dien-21,17-carbolacton erhalten, welche ohne weitere Reinigung in die nächste Stufe eingesetzt werden.

g) Eine Suspension von 160 g 3-Ethoxy-15β,16β-methylen-17α-pregn-3,5-dien-21,17-carbolacton in 6 l Aceton wird bei Raumtemperatur mit 26,5 ml Pyridin, 375 ml Wasser und 117,6 g Natriumacetat versetzt und auf 0°C abgekühlt. Dazu gibt man portionsweise 87,1 g N-Bromsuccinimid und rührt 30 Minuten bei 0°C nach. Anschließend engt man im Vakuum bei einer Badtemperatur von 20°C weitgehend ein. Der ausgefallene Feststoff wird mit 500 ml Dichlormethan gelöst, mit 8,8 l Ether verdünnt und zweimal mit je 2,2 l Wasser gewaschen. Nach dem Trocknen über Magnesiumsulfat engt man im Vakuum ein. Man erhält 174 g 6-Brom-15β,16β-methylen-3-oxo-17α-pregn-4-en-21,17-carbolacton als öliges Rohprodukt, das ohne Reinigung weiter umgesetzt wird.

Eine Lösung von 201 g 6-Brom-15β,16β-methylen-3-oxo-17α-pregn-4-en-21,17-carbolacton in 1,975 l Dimethylformamid wird mit 102,3 g Lithiumbromid und 86,5 g Lithiumcarbonat 1,5 Stunden bei 100°C gerührt. Nach dem Abkühlen fällt man in 18 l Eiswasser, saugt den ausgefallenen Niederschlag, ab, wäscht mehrmals mit Wasser nach und trocknet den Rückstand über Nacht im Trockenschrank. Das erhaltene Rohprodukt von 180 g wird durch eine Drucksäule an feinem Kieselgel mit Dichlormethan/Aceton (100/0-98:2) gereinigt.

Man erhält 106 g 15β,16β-Methylen-3-oxo-17α-pregna-4,6-dien-21,17-carbolacton als Öl, welches nach Umkristallisation aus Aceton-Diisopropyl ether bei 179,5°C schmilzt.

## Patentanspruch

Verfahren zur Herstellung von 15α-Hydroxy-4-androsten-3,17-dion durch mikrobiologische Hydroxylierung von 4-Androsten-3,17-dion, dadurch gekennzeichnet, daß man zur Fermentation Penicillium stoloniferum CBS P 102, Penicillium melinii CBS P 30 oder Fusarium oxysporum f. vasinfectum ATCC 7808 verwendet.

## Revendication

Procédé pour préparer l'hydroxy-15α androstène-4 dione-3,17 par hydroxylation microbiologique de l'androstène-4 dione-3,17, procédé caractérisé en ce qu'on utilise, pour la fermentation, Penicillium stoloniferum CBS P 102, Penicillium melinii CBS P30 ou Fusarium oxysporum f. vasinfectum ATCC 7808.

## Claim

Process for the manufacture of 15α-hydroxy-4-androstene-3,17-dione by the microbiological hydroxylation of 4-androstene-13,17-dione characterised in that *Penicillium stoloniferum* CBS P 102, *Penicillium melinii* CBS P 30 or *Fusarium oxysporum f. vasinfectum* ATCC 7808 are used for the fermentation.